Europäisches Patentamt

European Patent Office

Office européen des brevets

(11) Publication number: **0 513 446 A1**

# EUROPEAN PATENT APPLICATION

(21) Application number: **91303985.5**

(22) Date of filing: **02.05.91**

(51) Int. Cl.5: **A61B 10/00**

(43) Date of publication of application:
**19.11.92 Bulletin 92/47**

(84) Designated Contracting States:
**AT BE CH DE DK FR GB IT LI LU NL SE**

(71) Applicant: **Al-Sam, Salam Zahroon Dr.**
**Histopathology Department, Chelmsford and**
**Essex Hospital, London Road**
**Chelmsford CM2 0OH(GB)**

(72) Inventor: **Al-Sam, Salam Zahroon Dr.**
**Histopathology Department, Chelmsford and**
**Essex Hospital, London Road**
**Chelmsford CM2 0OH(GB)**

(54) **Tissue aspiration needle.**

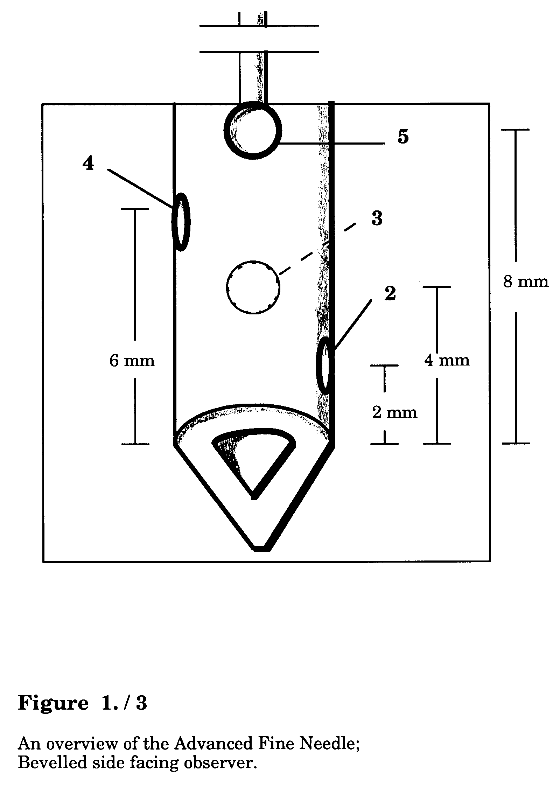

(57) An Advanced Fine Needle, gauge 21 - 22, 35 mm - 75 mm long with 4 additional channels , to expand the capacity of tissue aspiration for diagnosis and cancer screening, is shown in figures 1 - 3. The four additional intercalated channels are located at 2 mm, 4 mm, 6 mm, and 8 mm from the needle's tip, one of each is located on one of the four sides of the needle.

The channels open through holes of the same diameter to that of the needle's central canal. The edges of the holes are smooth and non injurious to the adjacent tissue.

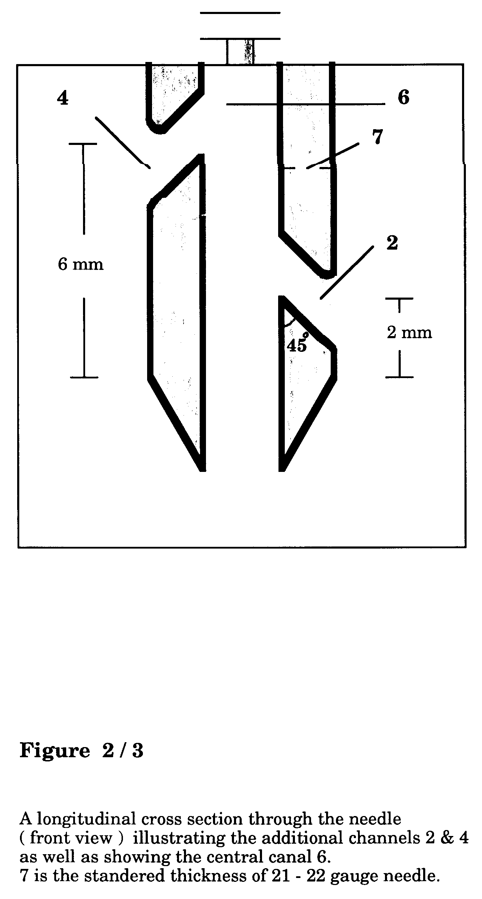

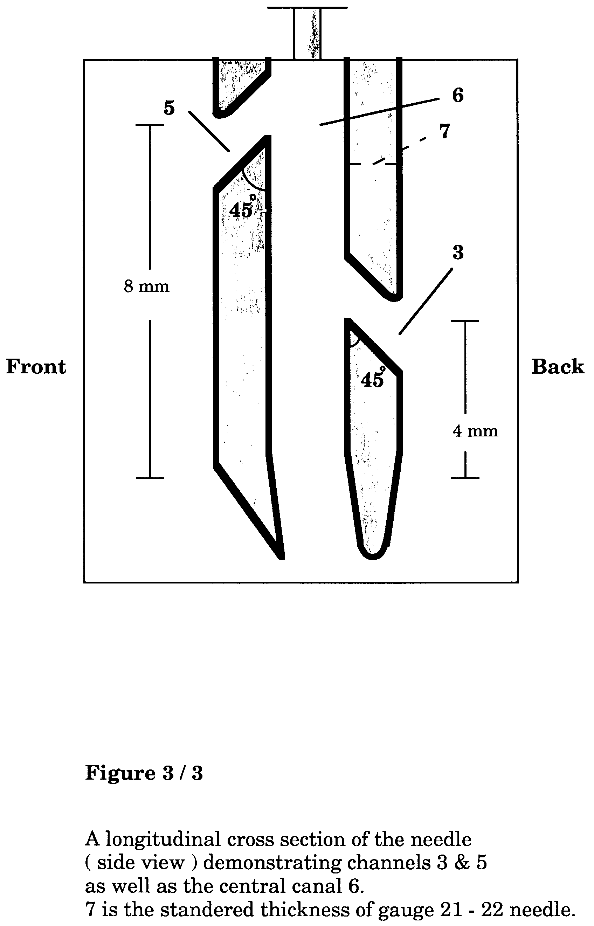

The new channels are preferably directed at 45° of the longitudinal axis measured from the distal end of the needle as shown in figure 2 and figure 3.

( PANEL F )

Figure 1. / 3

An overview of the Advanced Fine Needle;
Bevelled side facing observer.

Rank Xerox (UK) Business Services

This application relates to an Advanced Fine Needle for Tissue Aspiration.

Tissue aspiration for diagnostic purposes using a syringe and a needle has been in use for over 60 years around the world. At the present time, needle aspiration plays an important role in the breast cancer screening programme in England and elsewhere.

Unfortunately, one of the continuing problems remains the high rate of failure in obtaining tissue for diagnosis using the conventional 21 gauge - 22 gauge disposable needles. This failure rate ranges from 15% to 50% while small samples insufficient for cytological interpretation may also add to these figures.

Methods to reduce this high failure rate will be welcomed by the patient, the surgeon, the radiologist, the cytopathologist and the Government who spend thousands of pounds every year to keep this diagnostic and cancer screening programme going .

In this application a new advanced needle is described which may help to reduce the tissue aspiration failure rate and may also help to obtain larger samples from tissue for diagnostic and cancer screening purposes.

**According to the present invention, a 21 gauge to 22 gauge disposable needle 30 mm - 75 mm in length and 0.64 mm to 0.75 mm in external diameter should be used. It is supplied with four additional intercalated channels connected to the needle's central canal and opens to the needle's outer surface. These additional channels will provide a wider surface aspiration capacity which will help to minimize missing small pathological lesions deep within the tissue. It may also help to avoid failure to obtain samples in cases of blockage of the only hole available in the conventional needle by a blood clot, fibrous tissue or a calcified material.**

*A specific embodiment of the invention will now be described by way of example with reference to the accompanying drawings in which:-*

**Figure 1**   shows an overview of the new advanced disposable fine needle 21 gauge , 22 gauge of any acceptable length (30 - 75mm). This is a front view with the bevelled side facing the observer.The four additional holes( 2 - 5 ) are shown: hole 2 right 2 mm from needle tip. hole 3 back 4 mm from needle tip. hole 4 left 6mm from needle tip. hole 5 front 8 mm from needle tip.

**Figure 2**   Illustrates a longitudinal cross section through the needle (front view) to demonstrate the additional channels 2 and 4. The central canal ( 6 ) and the angle of direction of the new channels is preferably 45°. The needle's wall thickness ( 7 )is that of standered gauge 21 - 22 conventional needles.

**Figure 3**   Illustrates a longitudinal cross section through the needle ( side view, bevelled side to the left ) to demonstrate additional channels 3 and 5.

The four new holes should be distributed within the distal 8 mm of the needle ( close to the needle's tip ) and one of each will be located on one of the four sides of the needle as shown in the accompanying diagrams ( figures 1 - 3 ).

The diameter of each hole should be the same as that of the central canal of the needle ( 21 gauge - 22 gauge ).

The direction of the intercalated channels is preferably at 45° of the longitudinal axis of the needle measured from the distal end of the needle as shown in figures 2 and 3.

This is preferable to channels perpendicular to the main central canal of the needle and the purpose is to avoid side - splashing of tissue and eventual loss of the aspirated material during the process of expelling the tissue out of the needle on the slides.

The four additional channels should be intercalated to avoid weakening the distal end of the needle and to minimize the danger of snapping inside the tissue.

The rim of the new holes should be smooth ( rounded rather than sharp ) to avoid injuring the adjacent tissue and to minimize the bleeding and pain.

The existing method used in aspirating tissue by the conventional needle is expected to be more traumatic to the tissue as the needle should be moved in and out of the lesion many times to aspirate the maximum amount of material. With the advanced needle, described in this application, there is no need to move the needle back and forth inside the lesion and a gentle rotation of the needle is sufficient to expose the largest surface area of the pathological lesion to the multiple aspirating channels available.

This method may reduce both internal tissue bleeding and pain experienced by the patient.

**Claims**

1. **An Advanced Disposable Fine Needle for tissue aspiration, gauge 21 - 22, 30 - 75 mm long, and 0.64 - 0.75 mm outer diameter with 4 additional intercalated channels connected to the central canal of the**

**needle through holes on the four surfaces of the needle which are distributed within the distal 8 mm of the needle.**

2. An Advanced Disposable Fine Needle for tissue aspiration as claimed in claim 1 with 4 additional smooth edged holes on the surface located on the four sides of the needle at 2 mm, 4 mm, 6 mm, and 8 mm.

3. An Advanced Disposable Fine Needle for tissue aspiration as claimed in claims 1 and 2 with four additional channels preferably directed at 45° of the longitudinal axis, measured from the distal end of the needle.

4. An Advanced Fine Needle for tissue aspiration substantially as described herein with reference to figures 1 - 3 of the accompanying diagrams.

**Figure 1. / 3**

An overview of the Advanced Fine Needle;
Bevelled side facing observer.

**Figure 2 / 3**

A longitudinal cross section through the needle
( front view ) illustrating the additional channels 2 & 4
as well as showing the central canal 6.
7 is the standered thickness of 21 - 22 gauge needle.

**Figure 3 / 3**

A longitudinal cross section of the needle
( side view ) demonstrating channels 3 & 5
as well as the central canal 6.
7 is the standered thickness of gauge 21 - 22 needle.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-2 130 890 (DOWNS SURGICAL PLC) <br> * page 2, line 38 - line 50; figures 5,9 * <br> --- | 1 | A61B10/00 |
| Y | GB-A-2 017 499 (DR. EDUARD FRESENIUS) <br> * page 2, line 14 - line 16; figure 3 * <br> --- | 1 | |
| A | US-A-1 897 249 (CH. C. HONSAKER) <br> * page 2, line 45 - line 46; figures 3,7 * <br> --- | 2,3 | |
| A | EP-A-0 299 622 (MINNESOTA MINING AND MANUFACTURING COMPANY) <br> * column 5, line 13 - line 16; figure 3 * <br> ----- | 3 | |

TECHNICAL FIELDS SEARCHED (Int. Cl.5)

A61B
A61M

The present search report has been drawn up for all claims

| Place of search | Date of completion of the search | Examiner |
|---|---|---|
| THE HAGUE | 17 DECEMBER 1991 | SEDY R. |

EPO FORM 1503 03.82 (P0401)